# EUROPEAN PATENT SPECIFICATION

(11) **EP 1 878 383 B1**
(45) Date of publication and mention of the grant of the patent: **22.06.2011**
(21) Application number: 07020884.8
(22) Date of filing: 26.09.2003
(51) Int. Cl.: A61B 5/06, A61B 19/00, A61M 5/42

(54) **Detection of implanted injection port**
Ortung eines implantierten Injektionsports
Détection d'un port d'injection implanté

(30) Priority: 01.10.2002 US 260546
(43) Date of publication of application: 16.01.2008
(62) Divisional of application: 03799227.8
(73) Proprietor: Prosthesica AG, 6341 Baar (CH)
(72) Inventor: Forsell, Peter, 6300 Zug (CH)
(74) Representative: Höhfeld, Jochen

(56) References cited:
- EP-A- 0 646 381
- WO-A-01/47435
- WO-A-99/18879
- WO-A-2004/028623
- US-A- 4 222 374
- US-A- 4 784 646
- US-B1- 6 305 381

## Description

The present invention relates to an apparatus for detecting an injection port subcutaneously implanted in a patient.

It is important to locate the position of an injection port connected to a hydraulically operable surgical implant in a patient to be able to accurately inject a needle of a syringe through the membrane of the injection port (or simply for the purpose of locating the exact position of the injection port, or alternatively locating the membrane of the injection port), for supplying hydraulic fluid to or withdrawing hydraulic fluid from the implant. Such an injection port is typically arranged in connection (via a conduit) to a hydraulically adjustable implant, for example a food intake restriction device, implanted inside an obese patient.

Currently, a nurse or doctor locates an implanted injection port by simply feeling with the fingers on the patient's skin to find out where the injection port is situated. However, the nurse or doctor cannot know exactly where the injection needle should penetrate the skin, in order to penetrate the centre of the membrane of the injection port.

Document US-A-4 222 374 discloses a system for detecting a subcutaneously implantable injection port of a prosthetic device, the system comprising an implantable permanent ring-magnet and a magnetic detector for detecting the local permanent magnetic field emitted by the ring-magnet, the magnetic detector being movable externally along the patient's body to establish an injection position at the patient's skin in front of the implanted injection port. The injection port is part of a pump, and the permanent ring-magnet is mounted inside the pump surrounding the membrane of the injection port.

The object of the present invention is to provide an inexpensive system for accurately detecting an injection port subcutaneously implanted in a patient to enable an injection needle to safely penetrate the patient's skin directly into the centre of the injection port.

This object is obtained by a system as defined in claim 1. It comprises, inter alia, a magnetic device adapted to emit a local magnetic field, and a magnetic detector adapted to detect the local magnetic field emitted by the magnetic device. The magnetic device is designed to be subcutaneously implanted in the patient at the implanted injection port, and the magnetic detector is movable externally along the patient's body to establish an injection position at the patient's skin in front of the implanted injection port where the local magnetic field emitted by the magnetic device is detected by the magnetic detector.

Thus, the accurate injection position on the patient's skin in front of the injection port, which is hidden behind the skin, can be established using the apparatus of the present invention. With an injection needle placed in this injection position, it is an easy task to properly and safely insert the injection needle through the patient's skin directly into the injection port substantially in the centre thereof. The present invention is particularly advantageous to practise in obese people where an implanted injection port can be difficult to manually locate.

Generally, the magnetic detector includes a semiconductor circuit, preferably in the form of at least one Hall-element. By using one or more Hall-elements, a special type of semiconductor known in the art, it is easy to locate the centre of the magnetic field emitted by the magnetic device. The magnetic detector suitably comprises several Hall-elements grouped around a central point in a triangular or square configuration. For example, three Hall-elements may be arranged at the corners of an equilateral triangle. An important advantage is that the Hall-elements are able to detect even a weak magnetic field emitted from the magnetic device.

In accordance with the invention, the magnetic device is designed to be subcutaneously implanted in the patient at the implanted injection port to emit the local magnetic field through a portion of the patient's skin adjacent to the injection port, and the magnetic detector is movable externally along the patient's body to establish the injection position where the local magnetic field is detected by the magnetic detector. The magnetic device includes a ring-magnet to be positioned around the membrane of the injection port, so that an injection needle can be inserted through the ring-magnet and the membrane of the injection port. The magnetic detector may be adapted to emit a sound when detecting the local magnetic field. Alternatively, the movable magnetic detector may be provided with at least one diode adapted to emit light when the detector detects the local magnetic field, or be provided with a display adapted to indicate when the detector detects the local magnetic field.

The present invention can be used for detecting a wireless injection port subcutaneously implanted in a patient by providing a permanent ring-magnet capable of emitting a local magnetic field through the patient's skin, providing a magnetic detector adapted to detect the local magnetic field emitted by the magnetic device, subcutaneously implanting the permanent ring-magnet in the patient at the implanted injection port, moving the magnetic detector externally along the patient's body, and establishing an injection position at the patient's skin where the local magnetic field emitted by the magnet is detected by the magnetic detector. Then, an injection needle can be placed in the injection position where the local magnetic field has been detected to accurately insert the needle through the patient's skin directly into the injection port.

When practising the above detection method a semiconductor circuit, preferably comprising at least one Hall-element, may be used as the magnetic detector.

The invention is described in more detail in the following with reference to the accompanying drawings, in which
Figure 1 shows a connection diagram for a magnetic detector of the system according to the present invention,
Figure 2 schematically illustrates in a diagram the output of the magnetic detector positioned in front of a magnetic device of the system of the invention.
Figure 3 is a schematic view of an embodiment where the magnetic device is subcutaneously implanted in a patient, and the magnetic detector is movable externally along the patient's body,
Figure 4 is a schematic view of a hydraulically adjustable constriction device designed for treating reflux disease, urine incontinence, anal incontinence or obesity, and
Figure 5 illustrates an embodiment according to the present invention using Hall-elements as the magnetic detecting device.

Referring to the drawing figures, like reference numerals designate identical or corresponding elements throughout the several figures.

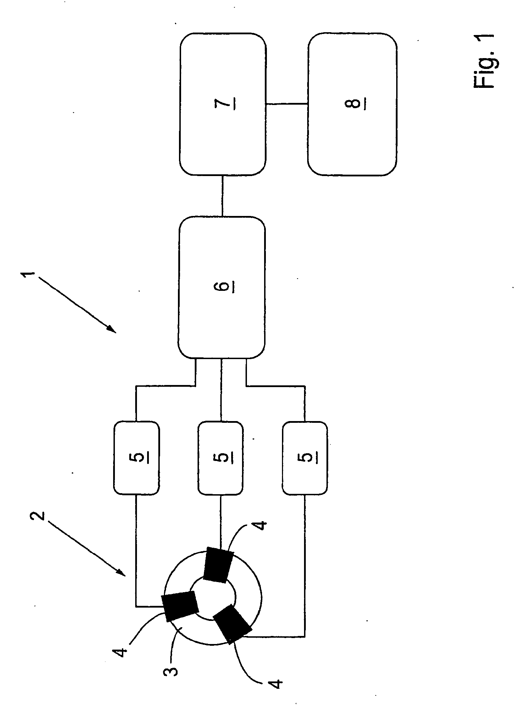

Figure 1 shows a connection circuit 1 for a magnetic detector 2 of the system according to the present invention. A magnetic device in the form of a permanent ring-magnet 3, is implanted in a patient's body. Located outside the body and positioned in front of the implanted ring-magnet 3 is magnetic detector 2, which includes three linear magnetic field sensors 4 grouped in a triangular configuration. Each sensor 4 includes a semiconductor circuit such as a Hall-element or the like. Sensors 4 are connected to signal-conditioning amplifiers 5, which in turn, are connected to an A/D-converter 6. A microprocessor 7 is connected to A/D-converter 6. To visually display the output signals of sensors 4, a display-device 8 is connected to microprocessor 7.

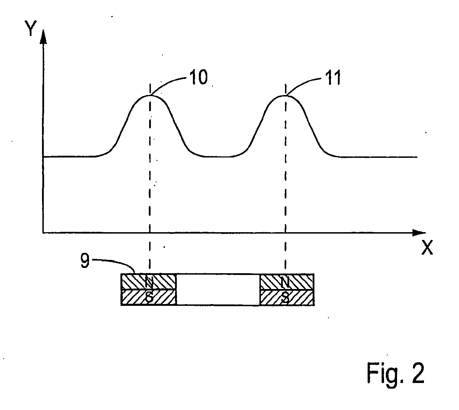

The graph shown in Fig. 2 illustrates, in principle, how the information obtained by detector 2 can be presented. On the X-axis in the graph is the position of detector 2 relative to the magnetic device. On the Y-axis is the combined output of sensors 4 of detector 2. Thus, the graph of Fig. 2 shows the position "X" of detector 2 relative to the magnetic device as a function of detector 2's output "Y". To illustrate this method of detecting, a magnetic device in the form of a ring-magnet 9 is shown relative to the graph of Figure 2. Ring-magnet 9 is shown in cross-section to show the positions of its magnetic north pole N and south pole S, respectively. Fig. 2 depicts the case where magnetic detector 2 (not shown in Fig. 2) has been centred in front of ring-magnet 9 and where all of the sensors 4 produce a maximum output, which is shown as peaks 10,11 in the graph of Fig. 2. Sensors 4 are connected (e.g., by connection circuit 1 shown in Fig. 1) to display device 8, which may display the graph shown in Fig. 2, or alternatively, a numeral result from the measurements taken by sensors 4.

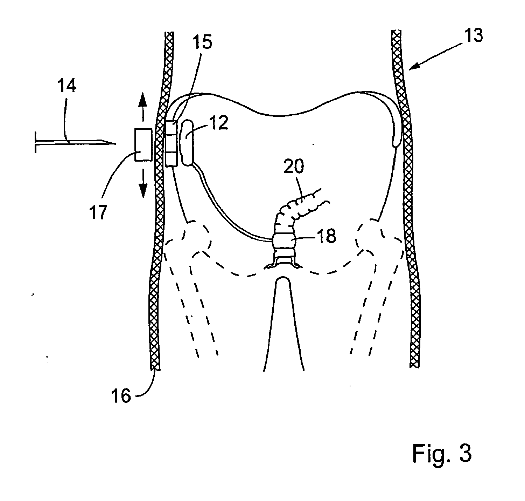

Fig. 3 shows an embodiment of the system of the present invention for detecting an injection port 12 subcutaneously implanted in a patient 13 suffering from anal incontinence to enable positioning of an injection needle 14 outside the patient's body for safe and accurate injection in the injection port 12. Injection port 12 is hydraulically connected to a hydraulically adjustable artificial sphincter 18 applied to the patient's rectum 20. The apparatus also includes a magnetic device in the form of a ring-magnet 15 subcutaneously implanted in the patient 13 around injection port 12. Magnetic device 15 emits a local magnetic field extending through a portion of the patient's 13 skin 16 adjacent to injection port 12. The apparatus further includes an external, separate magnetic detector 17 that may be manually moved along the patient 13's body to establish an injection position at the patient's skin where the local magnetic field emitted by magnetic device 15 is detected by magnetic detector 17. When this injection position has been established, injection needle 14 can be located in the same position to accurately insert the needle 14 through patient's skin directly into injection port 12.

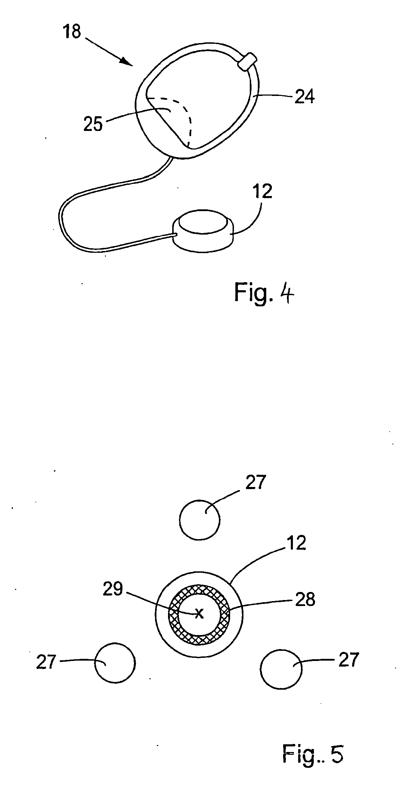

Fig. 4 shows an example of the artificial sphincter 18 shown in Fig. 3. Sphincter 18 includes a hydraulically adjustable constriction device 24 to be applied around the patient's rectum (not shown in Fig. 4). Constriction device 24 has a cavity 25 which can be inflated by supplying hydraulic fluid thereto, via implanted injection port 12, to close the rectum, and be deflated by withdrawing hydraulic fluid therefrom, to open the rectum. This type of constriction device may also be used as an artificial sphincter for treating patient's suffering from heartburn and reflux disease or urinary incontinence. Furthermore, constriction device 24 may be used for forming an adjustable stoma opening in the stomach or esophagus of an obese patient to treat obesity or for restricting the penile exit blood flow to treat an impotent patient.

Fig. 5 shows an advantageous design of the embodiment shown in Fig. 3, in which the external magnetic detector 17 includes three symmetrically arranged Hall-elements 27 which are grouped around a central point in a triangular configuration. The magnetic device is implanted and includes a ring-magnet 28 surrounding the centre 29 of the implanted injection port 12. When magnetic detector 17 is moved to a position in which Hall-elements 27 are placed symmetrically above and around ring-magnet 28, as illustrated in Fig. 5, magnetic detector 17 detects a maximum intensity of the magnetic field emitted by the implanted magnet 28, whereby the most accurate position where the injection needle 14 should be inserted into injection port 12 is established. Thus, the implanted magnetic detector 21 may include the three Hall-elements 27 and the external magnetic device 22 may include the ring-magnet 28.

Although the present invention has been described in terms of a particular embodiment, it is not intended that the invention be limited to that embodiment. Modifications of the embodiment will be apparent to those skilled in the art. The scope of the invention is defined by the claims that follow.

## Claims

1. A system comprising:
- an artificial sphincter (18) capable of being implanted inside a patient's body and comprising a hydraulically adjustable constriction device (24) having a cavity (25) inflatable by supplying hydraulic fluid thereto,
- an injection port (12) comprising a membrane and being capable of being subcutaneous implanted in the patient's body and hydraulically connected to the constriction device (24) for inflating and deflating the cavity (25) by supplying to and withdrawing from the cavity (25) hydraulic fluid via the injection port (12), and
- an injection port detection apparatus for detecting the injection port (12) when implanted,
said injection port detection apparatus comprising:
- a permanent ring-magnet (15) capable of being subcutaneously implanted around the membrane of the implanted injection port (12) subcutaneously implanted in a patient to emit a local permanent magnetic field through a portion of the patient's skin (16) adjacent to the injection port, and
- a magnetic detector (17) adapted to detect the local permanent magnetic field emitted by the ring-magnet,
- wherein the magnetic detector (17) is movable externally along the patient's body to establish an injection position at the patient's skin (16) in front of the implanted injection port where the local permanent magnetic field emitted by the ring-magnet is detected by the magnetic detector, whereby an injection needle can be placed in the established injection position, in order to insert the injection needle through the patient's skin and through the hole of the ring-magnet directly into the injection port substantially in the centre thereof.

2. A system according to claim 1, wherein the magnetic detector (17) comprises a semiconductor circuit.

3. A system according to claim 2, wherein the semiconductor circuit of the magnetic detector (17) comprises at least one Hall-element (27).

4. A system according to claim 3, wherein the magnetic detector (17) comprises several Hall-elements (27) grouped around a central point in a triangular or square-configuration.

5. A system according to any one of claims 1 to 4, wherein the magnetic detector (17) is provided with at least one diode adapted to emit light when the detector detects the local permanent magnetic field.

6. A system according to any one of claims 1 to 4, wherein the magnetic detector (17) is provided with a display adapted to indicate when the detector detects the local magnetic field.

## Patentansprüche

1. System, umfassend:
- einen künstlichen Schließmuskel (18), welcher in einen Körper eines Patienten implantierbar ist und eine hydraulisch anpassbare Verengungseinrichtung (24) mit einer Kavität (25) umfasst, welche durch Zuführen von hydraulischem Fluid zu dieser aufpumpbar ist,
- einen Injektionsport (12), welcher eine Membran umfasst und subkutan in den Körper des Patienten implantierbar ist und hydraulisch mit der Verengungseinrichtung (24) verbunden ist zum Aufpumpen und Entleeren der Kavität (25) durch Zuführen und Entnehmen von hydraulischem Fluid zu und aus der Kavität (25) über den Injektionsport (12), und
- eine Injektionsport-Detektionsvorrichtung zum Detektieren des Injektionsports (12), wenn er implantiert ist,
wobei die Injektionsport-Detektionsvorrichtung umfasst:
- einen permanenten Ringmagneten (15), welcher subkutan um die Membran des implantierten Injektionsports (12), welcher subkutan in einem Patienten implantiert ist, herum implantierbar ist, um ein lokales permanentes Magnetfeld durch einen zu dem Injektionsport benachbarten Teil der Haut (16) des Patienten hindurch zu emittieren, und
- einen Magnetdetektor (17), welcher eingerichtet ist, das von dem Ringmagneten emittierte lokale permanente Magnetfeld zu detektieren,
- wobei der Magnetdetektor (17) außen entlang des Körpers des Patienten bewegbar ist, um eine Injektionsposition auf der Haut (16) des Patienten vor dem implantierten Injektionsport zu ermitteln, wo das von dem Ringmagneten emittierte lokale permanente Magnetfeld von dem Magnetdetektor detektiert wird, wobei eine Injektionsnadel an der ermittelten Position platziert werden kann, um die Injektionsnadel durch die Haut des Patienten hindurch und durch das Loch des Ringmagneten hindurch direkt in den Injektionsport im Wesentlichen in dessen Mitte einzuführen.

2. System nach Anspruch 1, wobei der Magnetdetektor (17) einen Halbleiterschaltkreis umfasst.

3. System nach Anspruch 2, wobei der Halbleiterschaltkreis des Magnetdetektors (17) zumindest ein Hall-Element (27) umfasst.

4. System nach Anspruch 3, wobei der Magnetdetektor (17) mehrere Hall-Elemente (27) umfasst, welche um einen zentralen Punkt herum in einer Dreiecks- oder Rechteckskonfiguration gruppiert sind.

5. System nach einem der Ansprüche 1 bis 4, wobei der Magnetdetektor (17) mit zumindest einer Diode ausgestattet ist, welche eingerichtet ist, Licht zu emittieren, wenn der Detektor das lokale permanente Magnetfeld detektiert.

6. System nach einem der Ansprüche 1 bis 4, wobei der Magnetdetektor (17) mit einer Anzeige ausgestattet ist, welche eingerichtet ist, anzugeben, wenn der Detektor das lokale Magnetfeld detektiert.

## Revendications

1. Système comprenant:
- un sphincter artificiel (18) en mesure d'être implanté à l'intérieur du corps d'un patient, et comprenant un dispositif de constriction réglable par voie hydraulique (24) présentant une cavité (25) pouvant être gonflée en lui fournissant un fluide hydraulique,
- un orifice d'injection (12), comprenant une membrane et étant en mesure d'être implanté en sous-cutané dans le corps d'un patient et relié par voie hydraulique au dispositif de constriction (24) afin de gonfler et de dégonfler la cavité (25) en fournissant et retirant de la cavité (25) le fluide hydraulique via l'orifice d'injection (12), et
- un appareil de détection d'orifice d'injection destiné à détecter l'orifice d'injection (12) lorsqu'il est implanté,
ledit appareil de détection d'orifice d'injection comprenant :
- un aimant annulaire permanent (15) en mesure d'être implanté en sous-cutané autour de la membrane de l'orifice d'injection implanté (12) implanté en sous-cutané dans un patient, afin d'émettre un champ magnétique permanent local à travers une portion de peau du patient (16) adjacente à l'orifice d'injection, et
- un détecteur magnétique (17) propre à détecter le champ magnétique permanent local émis par l'aimant annulaire,
- dans lequel le détecteur magnétique (17) peut être déplacé de manière externe le long du corps du patient afin d'établir une position d'injection au niveau de la peau (16) du patient en face de l'orifice d'injection implanté où le champ magnétique permanent local émis par l'aimant annulaire est détecté par le détecteur magnétique, en sachant qu'une aiguille d'injection peut être placée dans la position d'injection établie, afin d'introduire l'aiguille d'injection à travers la peau du patient et à travers le trou de l'aimant annulaire directement dans l'orifice d'injection se trouvant pratiquement au centre de celui-ci.

2. Système selon la revendication 1, dans lequel le détecteur magnétique (17) comprend un circuit à semi-conducteurs.

3. Système selon la revendication 2, dans lequel le circuit à semi-conducteurs du détecteur magnétique (17) comprend au moins un élément de Hall (27).

4. Système selon la revendication 3, dans lequel le détecteur magnétique (17) comprend plusieurs éléments de Hall (27) groupés autour d'un point central dans une configuration triangulaire ou carrée.

5. Système selon l'une quelconque des revendications 1 à 4, dans lequel le détecteur magnétique (17) est doté d'au moins une diode propre à émettre une lumière lorsque le détecteur détecte le champ magnétique permanent local.

6. Système selon l'une quelconque des revendications 1 à 4, dans lequel le détecteur magnétique (17) est doté d'un affichage propre à indiquer le moment où le détecteur détecte le champ magnétique local.
